# EUROPÄISCHE PATENTSCHRIFT

(11) **EP 0 617 591 B1**
(45) Veröffentlichungstag und Bekanntmachung des Hinweises auf die Patenterteilung: **08.01.1997**
(21) Anmeldenummer: 92920381.8
(22) Anmeldetag: 01.10.1992
(51) Int. Cl.: A61B 17/38

(54) **KOAGULATIONSSONDE**
COAGULATION PROBE
SONDE DE COAGULATION

(30) Priorität: 19.11.1991 DE 9114438 U
(43) Veröffentlichungstag der Anmeldung: 05.10.1994
(73) Patentinhaber: WISAP GESELLSCHAFT FUR WISSENSCHAFTLICHEN APPARATENBAU MBH, D-8029 Sauerlach (DE)
(72) Erfinder: SEMM, Kurt, D-2300 Kiel (DE)
(74) Vertreter: Weber, Otto Ernst, Dipl.-Phys.
(86) Internationale Anmeldenummer: EP9202273
(87) Internationale Veröffentlichungsnummer: WO9306784

(56) Entgegenhaltungen:
- EP-A- 0 219 216
- DE-A- 2 929 398
- US-A- 3 698 394
- US-A- 4 672 962

## Beschreibung

Die Erfindung betrifft eine Koagulationssonde, insbesondere für eine Wärmekoagulation im Uterusbereich.

Koagulationsvorrichtungen, welche zur Blutstillung an Wundflächen dienen, weisen gewöhnlich ein Griffteil und eine Heizeinrichtung für eine aufheizbare und auf die Wundfläche aufsetzbare Druckfläche auf.

Eine in der DE-OS 29 29 398 beschriebene Vorrichtung wird im kalten Zustand auf die Wundflächen aufgesetzt, um Blut oder Gewebeflüssigkeit mechanisch zu verdrängen. Durch das kalte Aufsetzen und anschließende Erhitzen der Druckfläche soll ein Verkleben weitgehend verhindert werden. Für stark blutende Wundflächen ist die Druckfläche zur Verdrängung oder zur Absaugung des austretenden Blutes ausgebildet. Für Koagulationen im Uterusbereich ist diese Vorrichtung nicht geeignet.

Koagulationssonden für den Uterusbereich werden vor allem zur gezielten und dosierbaren Koagulation gutartiger Portioveränderungen und zur endozervikalen Therapie eingesetzt.

Bislang werden für diese Eingriffe Koagulationssonden verwendet, die eine zylindrisch ausgebildete Heizvorrichtung am distalen Ende eines stabförmigen Trägers aufweisen. Eine exakte Ausrichtung der Koagulationssonde, die notwendig ist, um die Beschädigung gesunden Gewebes zu vermeiden, ist hier jedoch aufwendiger und bedarf während des gesamten Koagulationsvorgangs einer ständigen präzisen Führung unter optischer Kontrolle.

Es ist daher **Aufgabe** der Erfindung, eine Koagulationssonde zu schaffen, mit der sich auf einfache Weise eine gezielte Koagulation, insbesondere im Portiobereich, durchführen läßt.

Diese Aufgabe wird durch eine Koagulationssonde mit den Merkmalen des Anspruchs 1 erfüllt. Vorteilhafte Weiterbildungen der Erfindung sind Gegenstand der Unteransprüche.

Erfindungsgemäß wird der Führungsstab, der auch für andere Geräte, z.B. für eine Stanze verwendet werden kann, unter optischer Kontrolle und Längsausrichtung des Uterus so tief in den Uterus eingeführt, daß er den Uterusboden perforiert. Nach dem Eingriff kann die Koagulationssonde bzw. der Hämostaser über den Führungsstab geschoben und an dem Führungsstab bis in den Portiobereich oder in den Uterus zur Koagulation gutartiger Veränderungen oder zur Hämostasierung hineingeführt werden. Durch die Führung der Koagulationssonde bzw. Hämostasters entlang des Führungsstabes ist eine genaue Positionierung im Portio- bzw. Unterusbereich möglich, wodurch eine Koagulation nicht zu behandelnder Gewebeteile vermieden wird.

Der Innendurchmesser des Grundkörpers des Koagulators ist vorzugsweise z.B. um 1 mm größer bemessen als der Innendurchmesser des Führungsstabs, damit der Wärmeübergang von der Heizeinrichtung am Grundkörper zum Führungsstab möglichst gering ist. Dies läßt sich ebenfalls erreichen, wenn am Innendurchmesser des Grundkörpers und/oder über den Umfang des Führungsstabes eine Führungseinrichtung aus wärmeisolierendem Material, z.B. aus Kunststoff oder Keramik, angeordnet ist. Diese Führungseinrichtung könnte z.B. die Form von Führungsringen haben, in denen der Führungsstab gleitbar geführt ist.

Während der hohlzylindrische Grundkörper vorzugsweise eine Länge von 20 bis 30 cm aufweist, ist die Heizeinrichtung von der distalen Seite her vorzugsweise über 1/4 bis 1/2 der Länge des Grundkörpers ausgebildet. Die Heizeinrichtung umgibt dabei den Grundkörper ebenfalls in der Form eines Hohlzylinders.

Die Heizeinrichtung besteht vorzugsweise aus einem Kupferrohr, das Längsnuten zur Aufnahme von Heizdrähten aufweist.

Die Heizeinrichtung kann auch durch zwei achsnormal zur Längsachse des Grundkörpers angeordnete Ringe bestehen, zwischen denen Heizdrähte aufgespannt sind. Die Heizdrähte sind vorzugsweise neusilberbeschichtet. Nach aussen hin ist die Heizeinrichtung teflonbeschichtet, um ein Anhaften von Gewebe zu vermeiden.

Auf dem Grundkörper des Koagulators kann eine Skalierung angeordnet sein, um die Einführtiefe der Koagulationssonde ablesen zu können.

Das am proximalen Ende des Grundkörpers angeordnete Griffteil der Koagulationssonde ist vorzugsweise 90° zur Längsachse des Grundkörpers in Art eines Pistolengriffs abgewinkelt. Hierdurch läßt sich die Koagulationssonde sicher führen.

Der elektrische Anschluß der Heizeinrichtung ist vorzugsweise im Mantel des hohlzylindrischen Grundkörpers und durch das Innere des Griffteils zu einem Koagulationsgerät geführt. Die Stromversorgung ist somit geschützt gegen eine Beschädigung von außen oder durch den Führungsstab.

Die Koagulationssonde ist aus rostfreiem Stahl, vorzugsweise V2A-Stahl hergestellt, und unempfindlich gegen eine chemische und/oder thermische Behandlung zur Sterilisierung des Geräts.

Zur Druckentlastung kann der Führungsstab von seinem proximalen Ende bis zu einem bestimmten Abstand zum distalen Ende eine Nut aufweisen, die ein Entweichen eines Überdrucks beim Einführen der Koagulationssonde in den Uterus ermöglicht. Der Außendurchmesser des Grundkörpers beträgt vorzugsweise 12 mm, während der Innendurchmesser 6,4 mm beträgt. Der Führungsstab hat einen Außendurchmesser von 6 mm und hat somit ein gewisses Spiel in der Innenbohrung des Grundkörpers. Im Bereich der Heizung ist der Durchmesser des Grundkörpers auf 13,5 mm erweitert. Das distale Ende des Grundkörpers mit der Heizeinrichtung ist vorzugsweise konisch oder abgerundet (sphärisch) ausgebildet. Bei einer Gesamtlänge des Grundkörpers von 21 cm beträgt die Länge der Heizeinrichtung am distalen Ende des Grundkörpers in etwa 75 mm, also ein Drittel der Länge des Grundkörpers.

Die Erfindung wird nachfolgend anhand der schematischen Zeichnung beschrieben. In dieser zeigen:
- Fig. 1: einen Längsschnitt durch eine Koagulationssonde mit Grundkörper, Heizeinrichtung und Griffteil;
- Fig. 2: einen Längsschnitt durch einen Führungsstab;
- Fig. 3: einen hinsichtlich Fig. 1 und 2 vergrößerten Teilausschnitt des Bereichs zwischen Grundkörper und Führungsstab mit einer Führungseinrichtung für den Führungsstab und
- Fig. 4: eine Koagulationssonde mit koaxial zum Grundkörper ausgebildetem Griffteil.

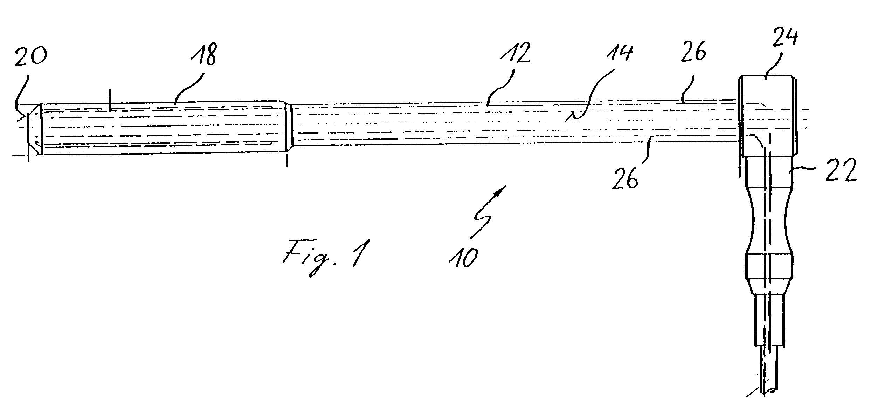

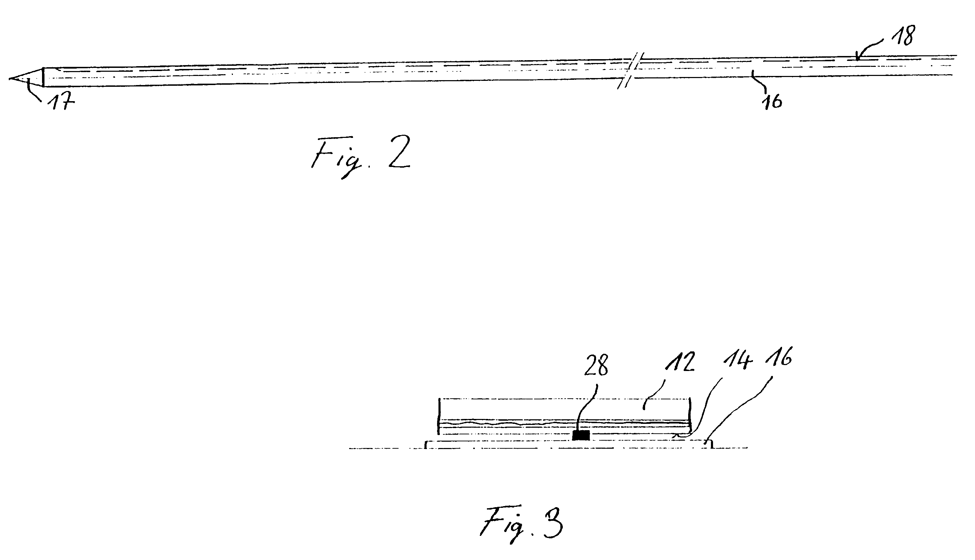

Die in Fig. 1 dargestellte Koagulationssonde 10 besteht aus einem hohlzylindrischen länglichen Grundkörper 12 mit einer inneren Bohrung 14 zur Aufnahme eines Führungsstabs 16 (Fig. 2). Am distalen Ende des Grundkörpers 12 ist eine Heizeinrichtung 18 angeordnet, die zylindrisch ungefähr das distale Drittel der Länge des Grundkörpers 12 umgibt.

Das distale Ende des Grundkörpers 12 bildet gleichzeitig das distale Ende der Heizeinrichtung 18 und ist zur Stirnseite 20 hin konisch zugespitzt.

Am proximalen Ende des Grundkörpers ist ein Griffteil 22 angeordnet, das mittels einer Manschette oder eines Flansches 24 an dem Grundkörper 12 gehalten ist. Die Zuleitungen 26 für die Heizeinrichtung 18 verlaufen im Inneren des Mantelrohres des Grundkörpers 12 und im Inneren des Griffteils 22, so daß keine Leitungen offenliegen und damit beschädigt werden können. Ein Kontakt der Leitungen 26 mit dem Körpergewebe ist ebenfalls ausgeschlossen. Die Leitungen 26 treten am offenen Ende des Griffteils 22 aus und führen in einem ummantelten Kabel zu einem Koagulationsgerät (nicht dargestellt).

Die Koagulationssonde ist insbesondere zur endovaginalen Therapie vorgesehen und läßt sich an dem Führungsstab 16 genau im Vaginal- oder Uterusbereich positionieren. Der Führungsstab 16 besitzt ungefähr eine Länge von 60 cm und weist eine parallel zur Längsachse verlaufende Nut 18 auf, um das Entweichen eines Überdrucks im Vaginal- oder Uterusbereich zu gestatten.

Die Nut 18 endet ungefähr 60 mm vor der distalen Spitze 17 des Führungsstabs 16, damit der Überdruck des Pneumoperitoneums nicht beim Durchstoßen des Uterusbodens entweicht.

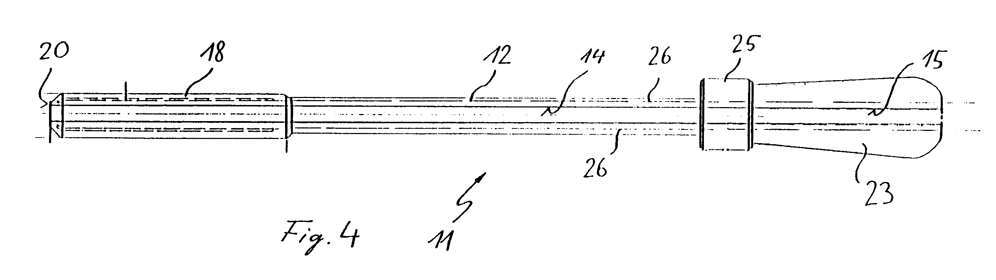

Fig. 3 zeigt einen Teilausschnitt des Grundkörpers 12 mit eingesetztem Führungsstab 16. In der Durchbohrung 14 für den Führungsstab 16 ist ein keramischer Führungsring 28 angeordnet, in dem der Führungsstab 16 axial verschiebbar geführt ist, wobei nur ein geringer Wärmeübergang von dem Grundkörper 12 mit der Heizvorrichtung 18 auf den Führungsstab 16 stattfindet. Hierdurch wird eine Koagulation von Gewebe an den Kontaktstellen zum Führungsstab 16 vermieden.

Das Gerät läßt sich insbesondere für eine Nachbehandlung nach der Aushülsung der Zervix verwenden. Zuerst wird der Führungsstab 16 in den Uterus eingeschoben, wobei der Uterus so ausgerichtet wird, daß die Spitze 17 des Führungsstabes 16 den Uterusboden in etwa zentral durchstößt. Anschließend wird uterines Gewebe mit einer hohlzylindrischen Stanze ausgestanzt, die mit einer distalen Schneide versehen ist. Durch diese Stanze wird ein zylindrischer Kanal aus dem uterinen Gewebe herausgelöst. Nach der Entfernung der Zervix-Stanze verbleibt der Führungsstab 16 in dem Uterus und die Koagulationssonde kann auf den Führungsstab aufgeschoben werden, wobei der Außendurchmesser der Heizeinrichtung mit dem Außendurchmesser der zuvor eingeführten Zervix-Stanze übereinstimmt. Die Koagulationssonde bzw. der Hämostaser wird nun soweit in den Uterus eingeführt, daß das gesamte im Uterus verbleibende Gewebe um den ausgestanzten Kanal mit der Heizeinrichtung in Kontakt kommt. Auf diese Weise werden offene Gefäße des verbleibenden uterinen Gewebes geschlossen und eine Blutstillung herbeigeführt.

Fig. 4 zeigt eine Koagulationssonde bzw. einen Hämostaser 11, der weitgehend identisch zu dem in Fig. 1 abgebildeten Hämostaser ist. Identische Teile sind hier mit identischen Bezugszeichen versehen. Die Koagulationssonde bzw. der Hämostaser 11 weist an ihrem bzw. seinem proximalen Ende eine Manschette 25 auf, an der ein axial verlaufender Griff 23 angeordnet ist. Dieser Griff 23 hat eine zentrische Durchbohrung 15, die mit der zentrischen Durchbohrung 14 des Grundkörpers 12 fluchtet. Die Leitungen 26 können entweder an der Manschette 25 radial oder am hinteren proximalen Ende des Griffes axial herausgeführt sein.

## Patentansprüche

1. Koagulstionssonde, insbesondere für den Uterusbereich, mit einer Heizeinrichtung (18) und mit einem Griffteil (22),
dadurch **gekennzeichnet,**
daß ein länglicher hohlzylindrischer Grundkörper (12) vorgesehen ist, welcher von einem Führungsstab (16) durchsetzbar ist,
daß das Griffteil (22) am proximalen Ende des Grundkörpers (12) und die Heizeinrichtung (18) am distalen Ende des Grundkörpers (12) angeordnet sind und
daß die Heizeinrichtung (18) den Grundkörper (12) umgibt oder in Verlängerung des Grundkörpers (12) angeordnet und ebenfalls hohlzylindrisch ausgebildet ist.

2. Koagulationssonde nach Anspruch 1,
dadurch **gekennzeichnet,**
daß das Griffteil (22) relativ zum Grundkörper (12) abgewinkelt oder axial dazu angeordnet ist.

3. Koagulationssonde nach Anspruch 1 oder 2,
dadurch **gekennzeichnet,**
daß die Abwinklung 90° beträgt.

4. Koagulationssonde nach einem der vorhergehenden Ansprüche,
dadurch **gekennzeichnet,**
daß der Innendurchmesser der Durchbohrung (14) des hohlzylindrischen Grundkörpers (12) größer ist als der Außendurchmesser des Führungsstabes (16).

5. Koagulationssonde nach einem der vorhergehenden Ansprüche,
dadurch **gekennzeichnet,**
daß die Heizeinrichtung (18) aus einem den Grundkörper (12) umgebenden Kupferrohr besteht, das Nuten für die Aufnahme von Heizdrähten aufweist.

6. Koagulationssonde nach einem der Ansprüche 1 bis 4,
dadurch **gekennzeichnet,**
daß die Heizeinrichtung (18) aus zwei relativ zur Längsachse des Grundkörpers (12) achsnormal angeordneten Ringen besteht, zwischen denen Heizdrähte verlaufen.

7. Koagulationssonde nach einem der Ansprüche 5 oder 6,
dadurch **gekennzeichnet,**
daß die Heizdrähte mit Neusilber beschichtet sind.

8. Koagulationssonde nach einem der vorhergehenden Ansprüche,
dadurch **gekennzeichnet,**
daß die Heizeinrichtung teflonbeschichtet ist.

9. Koagulationssonde nach einem der vorhergehenden Ansprüche,
dadurch **gekennzeichnet,**
daß die Leitungszuführungen (26) für die Heizeinrichtung (18) im Mantel des hohlzylindrischen Hohlkörpers (12) angeordnet sind.

10. Koagulationssonde nach einem der vorhergehenden Ansprüche,
dadurch **gekennzeichnet,**
daß der Grundkörper (12) eine wärmeisolierende Führung (28) für den Führungsstab (16) aufweist.

11. Koagulationssonde nach einem der vorhergehenden Ansprüche,
dadurch **gekennzeichnet,**
daß die Führung durch Zentrierringe (28) gebildet ist.

12. Koagulationssonde nach Anspruch 11,
dadurch **gekennzeichnet,**
daß die Zentrierringe (28) aus wärmeisolierendem Kunststoff oder Keramik bestehen.

## Claims

1. Coagulation probe, in particular for the uterine area, comprising a heating device (18) and a handle (22),
**characterized** in that
an elongated, hollow cylindrical main body (12) is provided which can be penetrated by a guide rod (16),
that the handle (22) is located at the proximal end of the main body (12) and the heating device (18) at the distal end of the main body (12) and
that the heating device (18) surrounds the main body (12) or is located in extension of the main body (12) and is also hollow cylindrically constructed.

2. Coagulation probe according to claim 1,
**characterized** in that
the handle (22) is offset or arranged axially with respect to the main body (12).

3. Coagulation probe according to claim 1 or 2,
**characterized** in that
the offset angle is 90°.

4. Coagulation probe according to one of the preceding claims,
**characterized** in that
the internal diameter of the bore (14) of the hollow cylindrical main body (12) is larger than the external diameter of the guide rod (16).

5. Coagulation probe according to one of the preceding claims,
**characterized** in that
the heating device (18) consists of a copper tube surrounding the main body (12) and having grooves for receiving of heating wires.

6. Coagulation probe according to one of the claims 1 to 4,
**characterized** in that
the heating device (18) consists of two rings arranged in axis-normal manner relative to the longitudinal axis of the main body (12) and that heating wires passe between the rings.

7. Coagulation probe according to one of the claims 5 and 6,
**characterized** in that
the heating wires are coated with nickel silver.

8. Coagulation probe according to one of the preceding claims,
**characterized** in that
the heating device is Teflon-coated.

9. Coagulation probe according to one of the preceding claims,
**characterized** in that
the leads (26) for the heating device (18) are located in the jacket of the hollow cylindrical main body (12).

10. Coagulation probe according to one of the preceding claims,
**characterized** in that
the main body (12) has a thermally insulating guiding device (28) for the guide rod (16).

11. Coagulation probe according to one of the preceding claims,
**characterized** in that
the guiding device is formed by centering rings (28).

12. Coagulation probe according to claim 11,
**characterized** in that
the centering rings (28) are made from thermally insulating plastic or ceramic.

## Revendications

1. Sonde de coagulation, en particulier pour la zone de l'utérus, avec un dispositif chauffant (18) et avec une partie formant poignée (22), ***caractérisée en ce qu***'un corps de base cylindrique creux allongé (12) est prévu, qui peut être traversé par une tige de guidage (16), ***en ce que*** la partie formant poignée (22) est située à l'extrémité proximale du corps de base (12) et le dispositif chauffant (18) à l'extrémité distale du corps de base (12), et ***en ce que*** le dispositif chauffant (18) entoure le corps de base (12) ou est situé dans prolongent du corps de base (12) et est également conformé en cylindre creux.

2. Sonde de coagulation selon la Revendication 1, ***caractérisée en ce que*** la partie formant poignée (22) forme un angle par rapport au corps de base (12) ou est disposée axialement à celui-ci.

3. Sonde de coagulation selon la Revendication 1 ou 2, ***caractérisée en ce que*** l'angle vaut 90°.

4. Sonde de coagulation selon l'une des Revendications précédentes, ***caractérisée en ce que*** le diamètre intérieur du perçage (14) du corps de base cylindrique creux (12) est supérieur au diamètre extérieur de la tige de guidage (16).

5. Sonde de coagulation selon l'une des Revendications précédentes, ***caractérisée en ce que*** le dispositif chauffant (18) est constitué d'un tube en cuivre entourant le corps de base (12), tube qui présente des rainures destinées à recevoir des filaments chauffants.

6. Sonde de coagulation selon l'une des Revendications 1 à 4, ***caractérisée en ce que*** le dispositif chauffant (18) est constitué de deux bagues situées perpendiculairement à l'axe longitudinal du corps de base (12), et entre lesquelles passent des filaments chauffants.

7. Sonde de coagulation selon l'une des Revendications 5 ou 6, ***caractérisée en ce que*** les filaments chauffants sont revêtus de maillechort.

8. Sonde de coagulation selon l'une des Revendications précédentes, ***caractérisée en ce que*** le dispositif chauffant est revêtu de Teflon.

9. Sonde de coagulation selon l'une des Revendications précédentes, ***caractérisée en ce que*** les conduites d'alimentation (26) du dispositif chauffant (18) sont logées dans l'enveloppe du corps de base cylindrique creux (12).

10. Sonde de coagulation selon l'une des Revendications précédentes, ***caractérisée en ce que*** le corps de base (12) présente un guidage thermo-isolant (28) pour la tige de guidage (16).

11. Sonde de coagulation selon l'une des Revendications précédentes, ***caractérisée en ce que*** le guidage est formé par des bagues de centrage (28).

12. Sonde de coagulation selon la Revendication 11, ***caractérisée en ce que*** les bagues de centrage (28) sont constituées d'une matière synthétique ou céramique thermo-isolante.
